# EUROPEAN PATENT APPLICATION

(11) **EP 3 502 232 A1**
(43) Date of publication of application: **26.06.2019**
(21) Application number: 17843556.6
(22) Date of filing: 22.08.2017
(51) Int. Cl.: C12M 3/00, C12N 15/115, C07K 14/78

(54) **CELL SCAFFOLD MATERIAL USING E-CADHERIN-BINDING NUCLEIC ACID APTAMER**

(30) Priority: 22.08.2016 JP 2016161807
(71) Applicant: Nissan Chemical Corporation, Tokyo 103-6119 (JP); The University Of Tokyo, Tokyo 113-8654 (JP)
(72) Inventor: FURUSHO Hitoshi, Chiba 2748507 (JP); YOSHIMOTO Keitaro, Tokyo 113-8654 (JP); MARUYAMA Ryo, Tokyo 113-8654 (JP)
(74) Representative: J A Kemp
(86) International application number: PCT/JP2017/029863
(87) International publication number: WO 2018/038076

(57) **Abstract**

A scaffold material for cell culturing using a nucleic acid aptamer that can be chemically synthesized, containing no animal-derived components, and having high biocompatibility is provided. A cell scaffold material containing an E-cadherin binding nucleic acid aptamer.

SEQUENCE LISTING

<110>  Nissan Chemical Industries, Ltd.
       The University of Tokyo

<120>  A material of cell scafold using E-cadherin binding aptomer

<130>  KP-16131

<160>  4

<170>  PatentIn version 3.5

<210>  1
<211>  34
<212>  DNA
<213>  Artificial Sequence

<220>
<223>  aptomer

<400>  1
ggggtggtgg gggatactgt gggggtggtg ggct                                    34


<210>  2
<211>  18
<212>  DNA
<213>  Artificial Sequence

<220>
<223>  primer

<400>  2
gcctgttgtg agcctcct                                                      18


<210>  3
<211>  18
<212>  DNA
<213>  Artificial Sequence

<220>
<223>  primer

<400>  3
cgcttattct tgtctccc                                                      18


<210>  4
<211>  70
<212>  DNA
<213>  Artificial Sequence

<220>
<223>  aptamer


<220>
<221>  misc_feature
<222>  (19)..(52)
<223>  n is a, c, g, or t

<400> 4
gcctgttgtg agcctcctnn nnnnnnnnnn nnnnnnnnnn nnnnnnnnnn nncgcttatt 60

cttgtctccc 70

## Description

### TECHNICAL FIELD

The present invention relates to a cell scaffold material containing a nucleic acid that may specifically bind to E-cadherin which is a cell adhesion factor.

### BACKGROUND ART

The proliferation and physiological function and differentiation potential of cells are known to vary depending on the binding of an adhesion protein present on the cell surface with the ligand of the adhesion protein (Non-Patent Document 1). Accordingly, if it would be possible to obtain a group of chemical modifiable molecules that strongly bind to the cell surface proteins and to provide a non-natural advanced binding form to cells, an establishment of a new methodology that could safely and conveniently improve cell functions without gene introduction can be expected.

The cell function is known to be heavily dependent on the culture environment. Above all, the adhesion of cells and substrate surface, and moreover, the adhesion between cells heavily influence the proliferation and differentiation of cells. For example, to provide a cell adhesion ability to the substrate surface, approaches such as modifying the substrate surface with proteins such as fibronectin and collagen, and moreover, with RGD peptide which is an integrin binding site of fibronectin, have been taken.

In the recent years, utilization of E-cadherin-immobilized substrate surface for maintaining the cell potency of stem cells at a single-layer state has been proposed. ES cells cultured in a gelatin-coated plate or a supporting cell layer (a feeder cell layer) form tightly aggregated colonies due to the cell-cell adhesion via E-cadherin (Non-Patent Document 2). Nagaoka et al., have shown that when mouse ES cells were cultured on a plate coated with a fusion protein of E-cadherin and the Fc domain of immunoglobulin (IgG) (ECad-Fc), there were no contact between cells and no colony was formed. Those cultured cells maintain all ES cell functions including pluripotency and germline transmission for differentiating into all three germ layer cells even after a long-term cultivation and had a high proliferative capacity than in the colony-forming state (Non-Patent Document 3).

In general, cell culture is carried out by using an animal-derived component. Normally, the culturing medium component contains about 5 to 10% of serum, and in order to further improve the survival rate, the growth rate, and the differentiation, a certain kind of protein is added. These sera and protein components are animal-derived components. Furthermore, human pluripotent stem (iPS) cells expected to be used as tools in regenerative medicine, medicinal efficacy, toxicity evaluation, and the like (Non-Patent Document 4), can be cultured using mouse embryonic-derived fibroblasts as feeder cells or scaffold materials derived from animal-derived components such as Matrigel (Mouse sarcoma-derived basement membrane matrix) and the like (Non-Patent Document 5). These cultures utilize cell-substratum adhesion, but not cell-cell adhesion.

Since these scaffold materials for human iPS cells contain various uncertain factors, safety concerns in clinical application is an issue, and since many animal-derived components are contained, it is necessary to take in consideration of the reproductivity among manufacturers and lot numbers. To elucidate the molecular mechanism of maintaining human iPS cells being undifferentiated or being differentiated, it is desirable to construct scaffold materials of non-animal-derived components (Xeno-free) which are all known compositions without containing uncertain factors.

Nucleic acid aptamers are a single-stranded DNA or RNA with a molecular recognition ability, and due to their structural diversity, many have already been reported to have high binding ability to various targets such as low molecules, proteins, cells, and thus, they have drawn attention as a novel molecular recognition element that could replace antibodies (Non-Patent Document 6). Nucleic acid aptamers are biopolymers having many advantages which antibodies do not, such as the following: they can be screened without using organisms, chemically modification can be introduced to an arbitrary location, nucleic acid aptamers can be easily linked to each other, and they have high thermal stability and preservability. Nucleic acid aptamers are materials that can be chemically synthesized, contain no animal-derived components, and have high biocompatibility.

Based on such features, making a nucleic acid aptamer as a scaffold material of a non-animal-derived component (Xeno-free) allows the maintenance of homogeneity of cells and tissues cultured *in vitro* and the safe use of them. For example, it is believed that safety concerns in clinical application which has been an issue in the existing scaffold materials for culturing human iPS cells and decrease in reproducibility among manufactures and lot numbers could be solved. Also, a scaffold material utilizing nucleic acid aptamers is expected to be able to systematically recover the cultured cells without damaging cell surface proteins by nuclease treatment. On the other hand, many nucleic acid aptamers that bind to various targets have been reported so far; however, no reports have been made as examples of nucleic acid aptamers alone inducing cell adhesion as well as applying as scaffold materials.

For the screening of a novel aptamer specifically binding to a protein or a cell, in vitro selection method is used as a most effective means. In particular, Systematic Evolution of Ligands by Exponential enrichment (SELEX) method is roughly divided into two steps: the selection of target nucleic acid molecules and the amplification of selected aptamers (for example, Patent Document 1). Repetition of the selection and amplification with an increased selection pressure results in obtaining nucleic acid fragments with high affinity. Moreover, in the recent years, various improvements have been made, and reports are made on such as methods with superior efficiency and selectiveness which can collect aptamers with fewer number of cycles. These aptamers have various advantages that cannot be found in antibodies such as the following: synthesis can be performed chemically and in a short period of time, the modification of molecules can be performed economically, the structure is simple, and hardly any immunogenicity is found, which are additional advantages other than the high-affinity and specificity to a target that are features of antibodies conventionally used in diagnosis and treatment.

For the nucleic acid consisting of the base sequence of SEQ ID NO: 1, its conformational structure and the binding ability to E-cadherin-Fc fusion protein have been reported (Non-Patent Document 8). However, the association with cell adhesion was not known.

### PRIOR ART DOCUMENTS

### Patent Documents

Patent Document 1: WO 91/19813

### Non-Patent Documents

Non-Patent Document1: Albelda, S. M. et al., The Faseb J. 1990, 4, 2868-2880.
Non-Patent Document 2: Larue, L. et al., Development 1996, 122, 3185-3194.
Non-Patent Document 3: Nagaoka, M. et al., PLoS ONE 2006, e15, 1-7.
Non-Patent Document 4: Takahashi, K. et al, Cell, 2007, 131, 861-872.
Non-Patent Document 5: Xu, C. et al., Nat. Biotechnol., 2001, 19, 971-974.
Non-Patent Document 6: Yang, L. et al., Adv. Drug Deliv. Rev. 2011, 63, 1361-1370.
Non-Patent Document 7: Jellinek et al., 1994, Biochemistry 33, 10450.
Non-Patent Document 8: Abstract of the 75th Symposium of the Japanese Society of Analytical Chemistry

### SUMMARY OF INVENTION

### Technical Problem

Based on such background, the present invention aims to provide a functional scaffold material that directly induces cell adhesion by using a substrate modified with a nucleic acid aptamer which is a material that can be chemically synthesized, that do not contain an animal-derived component, and that has a high preservability and biocompatibility.

### Solution To Problem

The present inventors have carried out dedicated research to solve the above-mentioned problem, thereby a nucleotide sequence having a specific binding ability to E-cadherin, one of the cell adhesion factors, has been specified by using SELEX methods that utilizes magnetic particles. Accordingly, the nucleic acid having the specific sequence has been found to be able to function as a specific aptamer to E-cadherin, and moreover, it has been found that the surface modified with the aptamer could directly induce cell adhesion, and thereby the present invention has been completed.

That is to say that, in one embodiment of the present invention, a cell scaffold material comprising a nucleic acid shown in SEQ ID NO: 1, which is prepared by adsorbing a nucleic acid aptamer specifically binding to E-cadherin onto a substrate is provided. Here, when these aptamers are RNAs, in the base sequences of SEQ ID NO: 1, T (thymine) is U (uracil). In a preferred embodiment of the present invention, the above-mentioned nucleic acid aptamer at its 5' or 3' end, may be linked to a primer recognition sequence, fluorescent labeling, to facilitate the detection, or may be linked to an amino group, a carboxy group, a vinyl group, biotin, avidin, or streptavidin, or to other specific binding tag peptides, to modify the substrate surface.

Furthermore, the present invention is also related to a method of preparing a support for cell culturing, in which the surface of the support is modified with a cell scaffold material containing an E-cadherin binding nucleic acid aptamer. Preferably, the E-cadherin binding DNA aptamer is a nucleic acid consisting of a base sequence shown in SEQ ID NO: 1. The E-cadherin binding DNA aptamer may comprise at least one chemical modification selected from the group consisting of a chemical substitution at a sugar chain moiety, a chemical substitution at a phosphate ester moiety, and a chemical substitution at a nucleic acid base moiety. The 5' or 3' end of the E-cadherin binding DNA aptamer may be modified with a functional group that can provide biotin, avidin, or streptavidin, or other covalent bond or non-covalent bond or may be modified with specific binding base sequence and tag peptides.

### Advantageous Effects Of The Invention

According to the present invention, a novel scaffold material using a nucleic acid aptamer could be provided. In other words, by using a nucleic acid aptamer that can specifically bind to E-cadherin, a scaffold environment for culturing that does not at all use a molecule which has a protein and an amino acid in its backbone structure may be provided. Furthermore, when recovering cells from the substrate surface, as it is not necessary to use a proteolytic enzyme, it would hardly damage the cells, and a scaffold material from where culture cells could be easily recovered is provided. Furthermore, only cells that largely express E-cadherin would be able to be easily separated, concentrated, and recovered from other cells in a form adhering to the substrate.

### BRIEF DESCRIPTION OF THE DRAWINGS

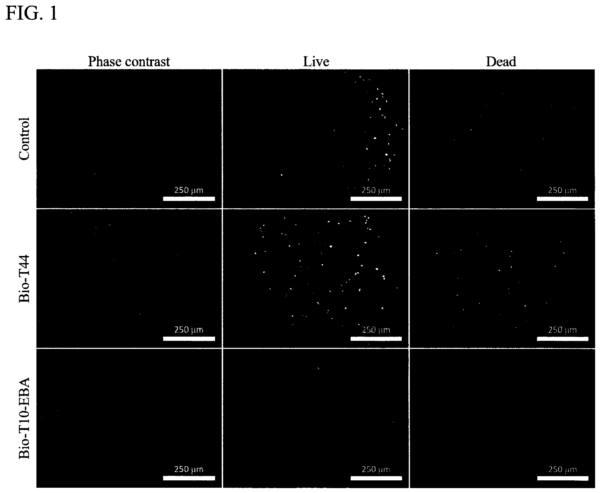

Fig. 1: A photographed image for verifying the live or death of cells on control, on a substrate coated with Bio-T44, and on a substrate bound with Bio-T10EBA of the present invention.

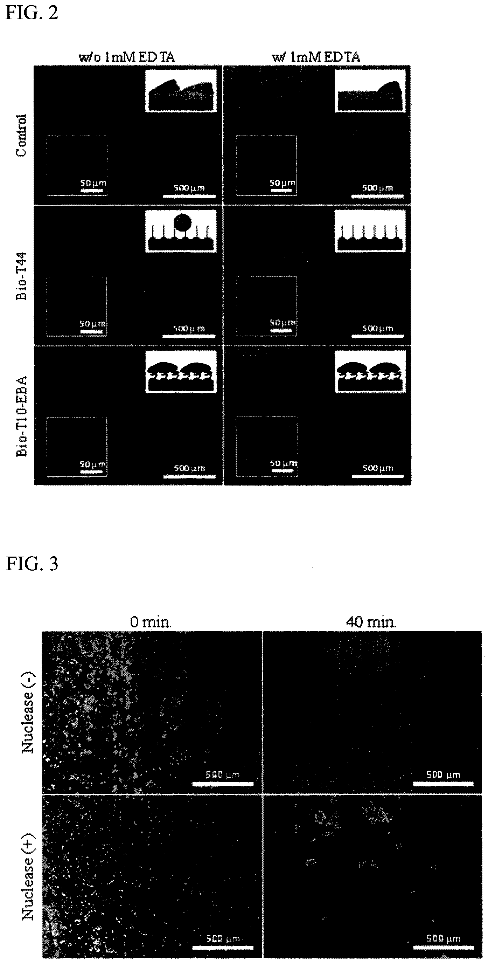

Fig. 2: A photographed image showing the adsorption state of cells before and after EDTA washing of the cells adsorbed on control, on a substrate coated with Bio-T44, and on a substrate bound with Bio-T10EBA of the present invention.
Fig. 3: A photographed image showing the recovery state of cells from a substrate bound with Bio-T10EBA, using nuclease.

### DESCRIPTION OF EMBODIMENTS

Hereinafter, the embodiments of the present invention are described. Nonetheless, the scope of the present invention would not be restricted to these descriptions, and those other than the exemplifications mentioned below can be performed with appropriate modifications which are made without departing from the spirit of the present invention.

### 1. Nucleic acid aptamers

In the present specification, a "nucleic acid aptamer" is a nucleic acid molecule having a short sequence of such as approximately 20 to 200 base length, for example, and it refers to a single-stranded nucleic acid molecule that can specifically recognize a molecule and substance that would be a target. The nucleic acid aptamer according to the present invention is a single-stranded nucleic acid molecule having a function specifically binding to E-cadherin.

A binding target of the nucleic acid aptamer of the present invention includes E-cadherin, preferably, an extracellular domain exposed on the cell surface of the amino acid sequence of E-cadherin, and more preferably, a conformational structure of the extracellular domain.
<SEQ ID NO: 1> 5' -GGGGTGGTGGGGGATACTGTGGGGGTGGTGGGCT-3'

The term "specific" in the present specification refers to a selective binding of a nucleic acid aptamer according to the present invention to E-cadherin. The binding specificity of an aptamer can be examined by comparing the binding of the aptamer to E-cadherin or cells expressing the E-cadherin on the cell membrane to the binding of the aptamer to an irrelevant protein or cells, under a predetermined condition. The nucleic acid aptamer according to the present invention is believed to be specific , if the binding to the E-cadherin is at least twice, at least five times, at least seven times, and preferably, at least ten times than the binding to the irrelevant protein or cells. An "irrelevant protein" is a protein that is different from E-cadherin, and the structure, function, and the like, of the protein shall be different from the E-cadherin. The binding form of the specific binding is not particularly limited; however, it can include covalent bond, ionic bond, hydrogen bond, electrostatic or hydrophobic interaction, and the like.

The nucleic acid aptamer of the present invention may be the above-mentioned SEQ ID NO: 1 in which one or more of nucleotides are substituted, deleted, or added, as long as the nucleic aptamer has the function to specifically bind to E-cadherin. Preferably, the nucleotides that are substituted, deleted, or added are 1 to 3 nucleotide(s), more preferably 1 or 2 nucleotide(s), and further preferably 1 nucleotide. Furthermore, as long as the nucleic acid aptamer function of SEQ ID NO: 1 is not hindered, another base sequence, nucleic acid aptamer, peptide, protein (enzyme and antibody) may be linked to the same sequence.

Moreover, when such substitution, deletion, or addition of a nucleotide is present, the sequence of the nucleic acid aptamer of the present invention can be a sequence which shows a identity of 90% or more, preferably 93% or more, or more preferably 96% or more, to each of the above-mentioned SEQ ID NO: 1 (hereinafter, they can be referred to as "homologs"). Here, when used in the present specification, the term "sequence identity" is used with a meaning generally recognized in the present technical field. The term typically refers to the number of nucleotides of the nucleic acid sequence of the subject matter matching to the nucleotides identical to the reference nucleic acid sequence, when examined by a sequence analysis program (for example, Karlin and Altschul, 1990, PNAS 87:2264-2268; Karlin and Altschul, 1993, PNAS 90:5873-5877) or a visual inspection.

When substituted by one or more nucleotides, the substitution can be carried out by a universal base. The term "universal base" refers to a meaning that is generally recognized in the present technical field. The term, in general, refers to a nucleotide base analogue that forms a base pair with each base of a standard DNA/RNA with hardly any difference and which can be recognized by an intracellular enzyme (for example, Loakes et al., 1997, J. Mol. Bio. 270: 426-435). Non-limiting examples of universal bases include, C-phenyl, C-naphthyl, and other aromatic derivatives, inosine, azole carbozamide, and nitroazole derivatives (3'-nitropyrole, 4-nitroindole, 5-nitroindole, 6-nitroindole, and the like) (Loakes, 2001, Nucleic Acids Res. 29: 2437).

Furthermore, there is no upper limit on the length of the nucleic acid aptamer according to the present invention as long as the nucleic acid aptamer has the function to specifically bind to E-cadherin, and as long as it does not hinder the nucleic acid aptamer function, the same sequence may be linked to another base sequence or nucleic acid aptamer. However, in view of the easiness of synthesis and problems of antigenicity and the like, the length of the nucleic acid aptamer in the present embodiment, for example, as its upper limit, is 200 bases or less, preferably 150 bases or less, or more preferably 100 bases or less.

When the number of the total bases is low, chemical synthesis and bulk production are easier, and there is a greater advantage in terms of cost. In addition, it is easily chemically modified, the safeness in the living body is high, and toxicity is low. The lower limit is provided as a number that is the same or more to the number of the bases in the above-mentioned SEQ ID NO: 1, i.e., 34 bases or more. A nucleic acid aptamer is preferably a single-stranded DNA (ssDNA); however, even in a case where a partial double-stranded structure is formed by taking a hairpin loop type structure, the length of that nucleic acid aptamer is calculated as the length of a single strand.

In a preferred embodiment, the nucleic acid aptamer of the present invention is any of the sequences selected from the group consisting of the nucleotide sequence shown in SEQ ID NO: 1, which can be a nucleotide sequence having a primer recognition sequence at each 5' and 3' end. In other words, in this case, the nucleic acid aptamer has the following nucleotide sequence:
5' -P₁-X-P₂-3'.
Wherein X is a nucleotide sequence selected from the group consisting of the nucleotide sequence shown in SEQ ID NO: 1 or is a sequence comprising 1 to 3 nucleotide substitutions, deletions, additions, in these sequences. P₁ and P₂ are a first and a second primer recognition sequence introduced for PCR amplification. Preferably, P₁ is GCCTGTTGTGAGCCTCCT (SEQ ID NO: 2) and P₂ is CGCTTATTCTTGTCTCCC (SEQ ID NO: 3).

The nucleic acid aptamer of the present invention may be chemically modified for an increase of the stability in the living body. Unlimited examples of such chemical modifications include a chemical substitution at the sugar chain moiety (for example, 2' -O methylation); a chemical substitution at the phosphate ester moiety (for example, phosphorothioation, amino group, lower alkyl amino group, and acetyl group), and a chemical substitution at a base moiety. Similarly, an additional base could be provided at the 5' or 3' terminal end. The length of such additional base is normally 5 bases or less. Additional bases may be a DNA or RNA; however, when DNA is used, there are cases that the stability of the aptamer may increase. Sequences of such additional bases include sequences such as ug-3' , uu-3' , tg-3' , tt-3' , ggg-3' , guuu-3' , gttt-3' , ttttt-3' , and uuuuu-3' , for example; however, they are not limited thereto.

### 2. Selection of nucleic acid aptamers

The nucleic acid aptamer of the present invention can be selected and obtained by using a well-known in vitro selection method in the present technical field. As preferable examples of such method, Systematic Evolution of Ligands by Exponential enrichment: SELEX method is used.

SELEX method includes multiple repetition of selection of a nucleic acid ligand (aptamer) binding to a target substance and an exponential amplification by polymerase chain reaction (PCR), thereby a nucleic acid molecule (a single-stranded DNA, RNA) having an affinity to the target substance is obtained.

Moreover, apart from these, the nucleic acid aptamer of the present invention can be selected and obtained using well-known methods in the present technical field.

As mentioned above, "in vitro selection method" is a method which selects aptamer molecules having an affinity to the target molecules and cells from a nucleic acid molecule pool containing random nucleotide sequences (e.g., DNA pool) and removes molecules which do not have an affinity. Furthermore, this allows the enrichment of an aptamer molecule having a strong binding ability by repeating the cycle which includes only amplifying the selected aptamer molecules using PCR methods and the like and performing the selection by affinity.

In particular, first of all, a single-stranded nucleic acid fragment comprising a random nucleotide sequence (base sequence) region of approximately 20 to 300 bases, preferably, 30 to 150 bases, or more preferably 30 to 100 bases, is prepared, in specific. The preparation of a group of randomized single-stranded nucleic acid fragments can be carried out by a method of chemical nucleic acid synthesis, a synthetic method using an automated nucleic acid synthesizer, and synthetic method using amplification such as PCR, and the combination of them. For the preparation of RNA from DNA, a single-stranded RNA library can be prepared by in vitro transcription with RNA polymerase such as T7RNA polymerase, for example, using a double-stranded DNA library as a template. As these single-stranded nucleic acid fragments allow PCR amplification, using those having base sequences that serve as primers at both ends is preferable.

The primer recognition sequence moiety may have an appropriate restriction enzyme site so that the primer moiety could be excised by a restriction enzyme after a PCR amplification. The length of the primer recognition sequence moiety to be used is not particularly limited; however, it is approximately 20 to 50, preferably 20 to 30 bases. Moreover, for allowing the separation of a single-stranded DNA after PCR amplification by electrophoresis and the like, labeling such as with radioactive labeling and fluorescent labeling at the 5' end can be carried out.

Next, nucleic acid fragments having the random nucleotide sequence obtained as above (library pool) can be mixed with a target molecule immobilized onto a support such as magnetic microparticles at an appropriate concentration ratio, and this is incubated under an appropriate condition. After incubation, the mixture is centrifuged to separate the nucleic acid fragment-target molecule complex from free nucleic acid fragments. Then, the supernatant portion of the separated solution is removed, and PCR reaction is carried out by using the single-stranded nucleic acid fragment collected from the obtained complex to perform the amplification of the target molecule binding nucleic acid sequence. Subsequently, a nucleic acid fragment which can form a complex with a target molecule can be made into a single strand according to the well-known methods in the present technical field. Such means include the separation utilizing the binding of streptavidin-immobilized magnetic particles with biotin, for example. Due to this, ssDNA having a cell binding ability can be separated from an amplified nucleic acid double-stranded chain, and moreover, unnecessary coexisting substance contained in the PCR reaction solution such as DNA polymerase could be removed. After this, a similar operation is carried out using the collected ssDNA as a library pool.

A series of operations from mixing with the above-mentioned nucleic acid fragment and the target molecule, separating the nucleic acid fragment bound with the target molecule, PCR amplification, and using the amplified nucleic acid fragment once again in the binding with the target molecule is carried out for a couple of rounds. By repeating the rounds, a nucleic acid molecule which more specifically binds with a target cell can be selected. The sequence analysis of the obtained nucleic acid fragment can be carried out by the well-known methods in the present technical field.

Accordingly, in one embodiment of the present invention, a method for screening a nucleic acid aptamer comprising the following steps is provided, the method comprises:
(a) contacting a target molecule with various kinds of single-stranded nucleic acid fragments, by which a complex of the target molecule and the single-stranded nucleic acid fragment is formed;
(b) removing a single-stranded nucleic acid fragment which does not form the complex;
(c) obtaining a group of candidate nucleic acid fragments comprising a single-stranded nucleic acid fragment obtained by the detachment of the complex;
(d) amplifying the group of candidate nucleic acid fragments;
(e) repeating the steps (a) to (d) several times;
(f) determining the base sequences of a group of candidate nucleic acid fragments after a predetermined number of repetition and specifying redundant nucleic acid fragments consisting of the base sequences detected redundantly;
(g) selecting the redundant nucleic acid fragments whose frequency of presence increases as the number of repetitions in step (d) increases, wherein the target molecule is one of the various kinds of molecules constituting a family, and wherein the method is characterized by repeating step (e) by alternatively using each molecule.

Moreover, it is preferred that the method comprises a counter selection step in which the complex is formed by using a target molecule immobilized on a support, the group of candidate nucleic acid fragments obtained in step (e) is contacted with a support on which a target molecule is not immobilized, and a candidate nucleic acid fragment which does not form a complex with the support is further selected.

It is further preferred that the base sequences of the redundant nucleic acid fragments would not contain a base sequence of nucleic acid fragment that only binds to a support on which the target molecule is not immobilized.

### 3. Support whose surface is modified with an E-cadherin binding aptamer

Supports include a molecular aggregate, substrate, a column filler for separation, various kinds of particles for separation and diagnosis, various materials of nano- to micro-level size, and the like. Molecular aggregates include, for example, micelles, vesicles, and the like made of polymers having hydrophobic and hydrophilic moieties. Furthermore, substrates include a polystyrene dish for cell culturing, a glass petri dish, a cover glass, a metal thin-film vapor-deposited substrate, and the like. Moreover, the cell surface cultured in a monolayer or three-dimensional form can also be modified with a nucleic acid aptamer. Particles for separation include particles with glass/silica as a base, and moreover, they include latex particles, magnetic particles, polystyrene particles, and the like. In addition, a support having a network structure such as a polymer gel is included.

In addition, the nucleic acid aptamer in the scaffold material of the present invention can generally be provided in an embodiment in which the nucleic acid aptamer is immobilized on a solid phase support.

For example, biotin can be bound to the end of the nucleic acid aptamer to form a complex, streptavidin can be immobilized on the surface of a solid phase support, and the nucleic acid aptamer can be immobilized on the surface of the solid phase support by the interaction of biotin and streptavidin. In addition to this, the immobilization may be carried out by means utilizing immobilization via a functional group that forms a covalent bond such as an amino group, a carboxy group, an aldehyde group, a maleimide group, a thiol group, and a vinyl group, and furthermore, electrostatic interaction or hydrophobic interaction. The kit may appropriately contain other reagents and the like as necessary, and for example, additives such as solubilizing agents, pH adjusting agents, buffer agents, and tonicity agents can be used, and the formulation amount can appropriately be selected by those skilled in the art.

### EXAMPLES

Hereinafter, the present invention will be further described in detail with reference to the Examples, but the present invention is not limited thereto.

### [Example 1] Selection of E-cadherin binding aptamer (EBA)

SELEX method using magnetic particles have been carried out to obtain a nucleic acid aptamer acting on the extracellular domain of E-cadherin. More specifically, a magnetic particle immobilized with a fusion protein (Recombinant Human E-Cadherin Fc Chimera, R&D SYSTEMS, Cat.No.:648-EC-100) of an extracellular domain and immunoglobulin (IgG) of E-cadherin was prepared (Pierce (Registered Trademark) Protein A Magnetic Beads, Thermo SCIENTIFIC, Cat. No.: 88845). Then, this was mixed with a random sequence which is an aptamer candidate sequence and the above-mentioned magnetic particles, which was then washed, eluted, amplified by PCR, made into a single strand, and this operation was repeated 6 rounds. By using magnets, the magnetic particles could be rapidly and conveniently recovered, and the washing operation step is made easier than in the methods using columns. To analyze the nucleic acid base sequence selected in the previous paragraph, sequence analysis was performed using IonPGM, the Next Generation Sequencer. The outline of the operation procedures has been shown below. (1) linking the tag sequence for analysis to PCR products of 4th, 5th, 6th rounds using PCR, (2) monoclonal amplification of each sequence by emulsion PCR, and (3) sequence analysis using the next generation sequencer. Using the CLC Genomics Workbench 6 manufactured by CLC Bio, alignment of the obtained sequence data (sequence number counts) was performed and alignment was performed using a software called Clustal X. The presence ratio of each sequence was calculated, the presence ratio to the number of rounds was plotted, and the sequence with increasing tendency was extracted.

For a DNA pool, an oligo DNA having the following sequence whose full length is 70 bases and the random sequence portion (N) is 34 bases was used.
DNA pool Random 34 (manufactured by Nihon Gene Research Laboratories)
Sequence: 5' -GCCTGTTGTGAGCCTCCT(N34)CGCTTATTCTTGTCTCCC-3' (SEQ ID NO: 4)
Length: 70 bases (random sequence corresponds to the 34 bases in the middle)
Molecular weight: 21391.3g/mol
Molar absorption coefficient: 630475L/mol·cm
The base sequences of both ends of the random sequence are primer sequences used in PCR. The immobilization of the fusion protein of the extracellular domain and immunoglobulin (IgG) of E-cadherin to the magnetic particle surface was made according to the instructions attached to the magnetic particles.

### Examples of SELEX using magnetic particles

Seventeen µL of protein immobilized magnetic particles was taken, washed well with buffer I (phosphate buffer, pH 7.4, Ca, Mg free, 2mM EDTA, 0.1% HSA) with the use of magnets. To this, 20 µL of a DNA pool prepared at a concentration of 10 µM was added and mixed at room temperature for 30 minutes. After this, target binding DNA and target non-binding DNA were separated by carrying out the washing for 3 times in buffer I by using a magnet. Finally, the solution was substituted with 50 µL of TE buffer, heated at 95°C for 10 minutes, and the DNA adsorbed on protein immobilized magnetic particles was detached. As sense strand DNA having a protein binding ability is contained in the recovered supernatant, and this was amplified by using PCR methods. The amplified DNA is of a double-stranded structure, and as a primer sequence forming an anti-sense strand is modified with biotin, only the target sense strand of this double strand was purified and recovered using a streptavidin immobilized magnetic particles. This recovered single-stranded DNA was once again mixed with a protein immobilized magnetic particles and the above-mentioned procedures were repeated. First, the above-mentioned procedures were performed on the E-cadherin extracellular domain immobilized magnetic particles, and subsequently, the above-mentioned operations were performed on the immunoglobulin (IgG) fusion protein immobilized magnetic particles. This was considered as one cycle, and when a total of 6 cycles were carried out, the obtained DNA was analyzed using a Next Generation Sequencer (Ion Torrent PGM).

### Examples of sequence data analysis

The sequence data obtained in the fourth, fifth and sixth cycles was analyzed using a software (CLC). Furthermore, these sequence data were exported, and a file combining these into one was prepared, and an alignment was performed using a software (Clustal X and the like). Based on the obtained analysis results, sequences which showed an increased presence ratio in the course of 4R to 6R were selected. The base sequences of the DNA aptamers selected by such are indicated below.
<SEQ ID NO: 1> 5' -GGGGTGGTGGGGGATACTGTGGGGGTGGTGGGCT-3'

### The binding ability assessment using a surface plasmon resonance sensor

E-cadherin binding nucleic acid aptamer was immobilized onto one of the two channels on the chip, and bio-dT44 (a nucleotide consisting of 44 thymines) was immobilized on the other channel as a reference. After this, the two channels were linked, and then, E-cadherin-Fc was injected at a gradient concentration at a flow rate of 20 µL/min for 210 seconds (DPBST (running buffer, pH 7.4): DPBS (Wako, Cat. No.: 041-20211) with 0.27 g of Na₂HPO₄ • 12H₂O and Tween 20 0.05%). E-cadherin-Fc of each concentration was injected, switched to a running buffer and left for 10 minutes. Then, 50 mM NaOH + 1M NaCl solution was allowed to flow alternatively, and by this, the adsorption of E-cadherin-Fc on the sensor surface was washed. By repeating this, it is possible to obtain data of different E-cadherin-Fc concentrations in a single chip. IgG-Fc was also injected as a control; however, no response change was confirmed. At the data processing stage, data of the channel in which bio-dT44 was immobilized was used as a reference, and the dissociation constant was analyzed using the subtracted sensorgram.

### Preparation of the E-cadherin-conjugated DNA aptamer immobilized substrate and the assessment of cell adhesion

Each well of Immobilizer Streptavidin plate was washed 3 times with 300 µL of DPBST. Then, 100 µL of Bio-T10-EBA (500 nM) and Bio-T44 (500 nM) were added, and this was incubated at room temperature for 1 hour. After incubation, immobilization of nucleic acid aptamer was completed by washing each well for three times with 300 µL of DPBST.

A549 cells were seeded on the prepared substrate, and the survival rate of the cells on the present substrate was evaluated by the following procedure. Upon confluence, A549 was washed twice with 2 mL of DPBS (-), 10 mL of 1 mM EDTA-PBS (-) was added and incubated at 37 °C for 10 minutes to detach the cells. The prepared plates were seeded so that they would become 1 × 10⁴ cells/well. After 24 hours, the medium was removed and the Live-Dead Cell staining Kit (BioVision, Cat. No.: # K501-100) was used for staining, and then a fluorescence observation image was taken with OLYMPUS IX51. Based on this image, live cells and dead cells adhering on the EBA immobilized substrate were counted and quantified. As a result of this, 92% were live cells.

Other substrates were prepared, and the assessment of cell adhesiveness of the present substrate was carried out as follows. A surface immobilized with Bio-T44 (500 nM) was prepared by the same procedure as Bio-T10-EBA (500 nM). After seeding, A549 that reached confluence was washed twice with 2 mL of DPBS(-) (Wako, Cat. No.: 041-20211), and then 10 mL of 1 mM EDTA-PBS (EDTA Solution (BioVision, Cat. No.: 2103-100), DPBS (Wako, Cat. No.: 041-20211) were added and incubated at 37 °C for 10 minutes to detach the cells. Seeding was carried out so that it would become an oligo-coated 96-well plate at 3 × 10⁴ cells/well. After 1 hour, this was washed with 1 mM EDTA-DPBS.

By washing with EDTA, the cells were detached in the substrate uncoated with nucleic acid and in the substrate coated with Bio-T44. On the other hand, in the wells coated with Bio-T10-EBA, cells hardly got detached. it is believed that cells are detached by washing with EDTA, since ions such as calcium are important for the cell-substrate adhesion and the cells remained to be adhered as such ions are relatively unimportant for binding between aptamers and cells.

### Recovery of cells adhered to E-cadherin binding DNA aptamer immobilized substrate

A549 cells were seeded on Bio-T10-EBA-immobilized substrate at 1 × 10⁵ cells/well. After 1 hour, after confirming the adhesion of the cells on the Bio-T10-EBA immobilized substrate, the medium was removed, and then 100 µL of the mixed solution of Nuclease BAL-31 (New England Biolabs, Cat. No.: M0213S) and reaction buffer (composition: 600 mM NaCl, 12 mM CaCl₂, 12 mM MgCl₂, 20 mM Tris-HCl, 1mM EDTA, pH 8.0) or a reaction buffer alone were added and incubated at 37 °C for 40 minutes. After that, the image was captured by OLYMPUS IX51 and the image was obtained.

A549 adhered on the Bio-T10-EBA immobilized substrate was detached by incubating with a solution containing nuclease. It is believed that it is possible to recover cultured cells without damaging cell surface proteins by nuclease treatment.

As is obvious from these results, it is shown that the substrate on which the DNA aptamer of the present invention is immobilized functions as a cell scaffold material.

### INDUSTRIAL APPLICABILITY

The nucleic acid aptamer of the present invention is featured that it is easily chemically modified depending on the means for detection, such as fluorescent labeling, it has a high preservability and stability, and there is no need to use a proteolytic enzyme at the time of recovery, and thus, in the preparation of a functional culture plate that can improve the cell adhesion ability, it has greater advantages than utilizing a protein. Furthermore, as chemical synthesis can be easily carried out, a large-scale culturing device such as for antibodies is not required, and for the advantage that it can reduce the trouble and cost for preparation as well as it can continuously supply products with uniform performance, its industrial utility value is very high.

## Claims

1. A cell scaffold material comprising an E-cadherin binding nucleic acid aptamer.

2. The cell scaffold material according to claim 1, wherein the E-cadherin binding nucleic acid aptamer is a nucleic acid consisting of the base sequence shown in SEQ ID NO: 1.

3. A support for culturing cells whose surface is modified with the cell scaffold material according to claim 1 or 2.

4. The cell scaffold material according to claim 1 or 2, wherein the aptamer comprises at least one chemical modification selected from the group consisting of a chemical substitution at a sugar chain moiety, a chemical substitution at a phosphate ester moiety, and a chemical substitution at a nucleic acid base moiety.

5. The cell scaffold material according to claim 1 or 2, wherein the 5' or 3' end of the aptamer is modified with a functional group that can provide biotin, avidin, or streptavidin, or other covalent bond or non-covalent bond, or is modified with a specific binding base sequence and a tag peptide.
